# EUROPÄISCHE PATENTSCHRIFT

(11) **EP 0 897 297 B1**
(45) Veröffentlichungstag und Bekanntmachung des Hinweises auf die Patenterteilung: **12.11.2003**
(21) Anmeldenummer: 98904057.1
(22) Anmeldetag: 13.01.1998
(51) Int. Cl.: A61K 6/00

(54) **DENTALADHÄSIVE**
DENTAL ADHESIVE
ADHESIF DENTAIRE

(30) Priorität: 18.01.1997 DE 19701599
(43) Veröffentlichungstag der Anmeldung: 24.02.1999
(73) Patentinhaber: Heraeus Kulzer GmbH & Co.KG, 63450 Hanau (DE)
(72) Erfinder: FINGER, Werner, D-41469 Neuss (DE)
(74) Vertreter: Kühn, Hans-Christian
(86) Internationale Anmeldenummer: EP9800142
(87) Internationale Veröffentlichungsnummer: WO98031325

(56) Entgegenhaltungen:
- EP-A- 0 282 280
- EP-A- 0 684 033
- EP-A- 0 712 621
- EP-A- 0 787 477
- WO-A-96/23480

## Beschreibung

Die Erfindung betrifft eine Zubereitung zur Verwendung als Adhäsivkomponente, vorzugsweise für die Behandlung der Zahnhartsubstanz und von Dentallegierungen.

Ein besonders gravierendes Problem in der konservierenden Zahnheilkunde besteht in der dauerhaften, randspaltfreien Verklebung von Kunststoff-Füllungsmaterialien mit der Zahnhartsubstanz (Dentin und Schmelz). Im Dentalbereich werden aushärtbare Materialien als Füllungsmaterialien bei Zahnreparaturen verwendet. Als aushärtbare Materialien werden im allgemeinen Füllungsmaterialien auf Acrylatbasis bevorzugt, die durch radikalische Polymerisation in der Zahnkavität ausgehärtet werden. Ein Nachteil dieser Materialien besteht darin, daß sie während der Aushärtung schrumpfen und so zur Bildung von Randspalten zwischen der Kavitätenwandung und der Zahnfüllung beitragen. Die Kunststoff-Füllungen haben den zusätzlichen Nachteil, daß sie schlecht am Dentin haften bleiben.

Um die Bindung an die Zahnhartsubstanz zu verbessern, kann man sogenannte Dentaladhäsive einsetzen. Dabei werden Dentaladhäsive bevorzugt, die sowohl am Dentin als auch am Schmelz gute Bindungsfestigkeiten ergeben. Wirksame Formulierungen enthalten im allgemeinen eine Vielzahl von Komponenten. So wird in DE-A-38 28 170 ein Beschichtungsmittel für kollagenhaltige Materialien beschrieben, das aus
a) Aldehyd,
b) einem wasserlöslichen Monomer mit aktivem Wasserstoff,
c) einem wasserunlöslichen Monomer mit zwei oder mehreren polymerisierbaren Doppelbindungen,
d) einem Fotoinitiator,
e) Wasser,
f) einem Löslichkeitsvermittler und/oder Dispergator und
g) bekannten Zusätzen
besteht.

Mit diesem Beschichtungsmittel lassen sich Bindungsfestigkeiten von 11,8 bis 19 N/mm² an Dentin und von 12,6 bis 17 N/mm² an Schmelz erreichen.

EP 712 621 A1 betrifft zweischichtige Kunststoffüberzüge mit Polyacrylnitril/Polyacrylat-Copolymer in der einen und Polyurethan in der anderen Schicht. Die Polyurethankomponenten sind isocycanatfreie Einbrennharze, welche bei Temperaturen über 100°C Additions- und Kondensatinsreaktionen eingehen, also keiner radikalischen Photopolymerisation unterliegen.

WO 96/23480 A1 beschreibt einkomponentige sogenannte "self etch" Adhäsive, enthaltend multifunktionelle polymerisierbare Verbindungen mit einer Phosphat- und mindestens drei Acyrlyatgruppen, gegebenenfalls weitere polymerisierbare Acrylatverbindungen, Lösungsmittel und Photoinitatoren. Bindungsfestigkeiten an Dentin von 12 bis 13.9 MPa werden berichtet.

Die Mehrzahl der bekannten Dentaladhäsive enthält als funktionelles Monomer Hydroxyethylmethacrylat, eine Verbindung, die eine stark sensibilisierende Wirkung zeigt (Katsuno K, Manabe A, Itoh K et al. Contact dermatitis caused by 2-HEMA and GM dentin primer solutions applied to guinea pigs and humans. Dent Mater J 1996; 15: 22-30).

Es wurde nun eine Zubereitung gefunden, die frei von Hydroxyethylmethacrylat ist, wenige Komponenten enthält, sehr einfach angewendet werden kann und sehr hohe Bindungsfestigkeiten an Schmelz, Dentin, Keramik, Metallen und Metall-Legierungen ermöglicht.

Die erfindungsgemäße Zubereitung ist dadurch gekennzeichnet, daß sie aus
a) 10 - 47,5 Gew.-% Urethandi(meth)acrylat,
b) 2,5 - 40 Gew.-% Methacryloyloxy-Gruppen aufweisenden Estem aromatischer Tri- oder Tetracarbonsäuren oder der entsprechenden Anhydride,
c) 25 - 75 Gew.-% eines flüchtigen, mit Wasser mischbaren Lösungsmittels,
d) 0,01 -2,5 Gew.-% Fotoinitiator und
e) 0-40 Gew.-% an sich bekannter Zusätze
besteht.

Der Anteil an Urethandi(meth)acrylaten beträgt 10 - 47,5 Gew.-%, vorzugsweise 25 - 45 Gew.-%. Die erfindungsgemäßen Urethandi(meth)acrylatesind Umsetzungsprodukte von Diisocyanaten mit Hydroxyalkyl(meth)acrylaten. Die Urethandi(meth)acrylate können sich von aliphatischen, verzweigt-aliphatischen, cyclo-aliphatischen oder aromatischen Diisocyanaten ableiten. Bevorzugt werden die Umsetzungsprodukte von aliphatischen, verzweigt-aliphatischen und cyclo-aliphatischen Diisocyanaten. Beispielhaft seien die folgenden Urethandiacrylate (UDA) und Urethandimethacrylate (UDM) genannt:

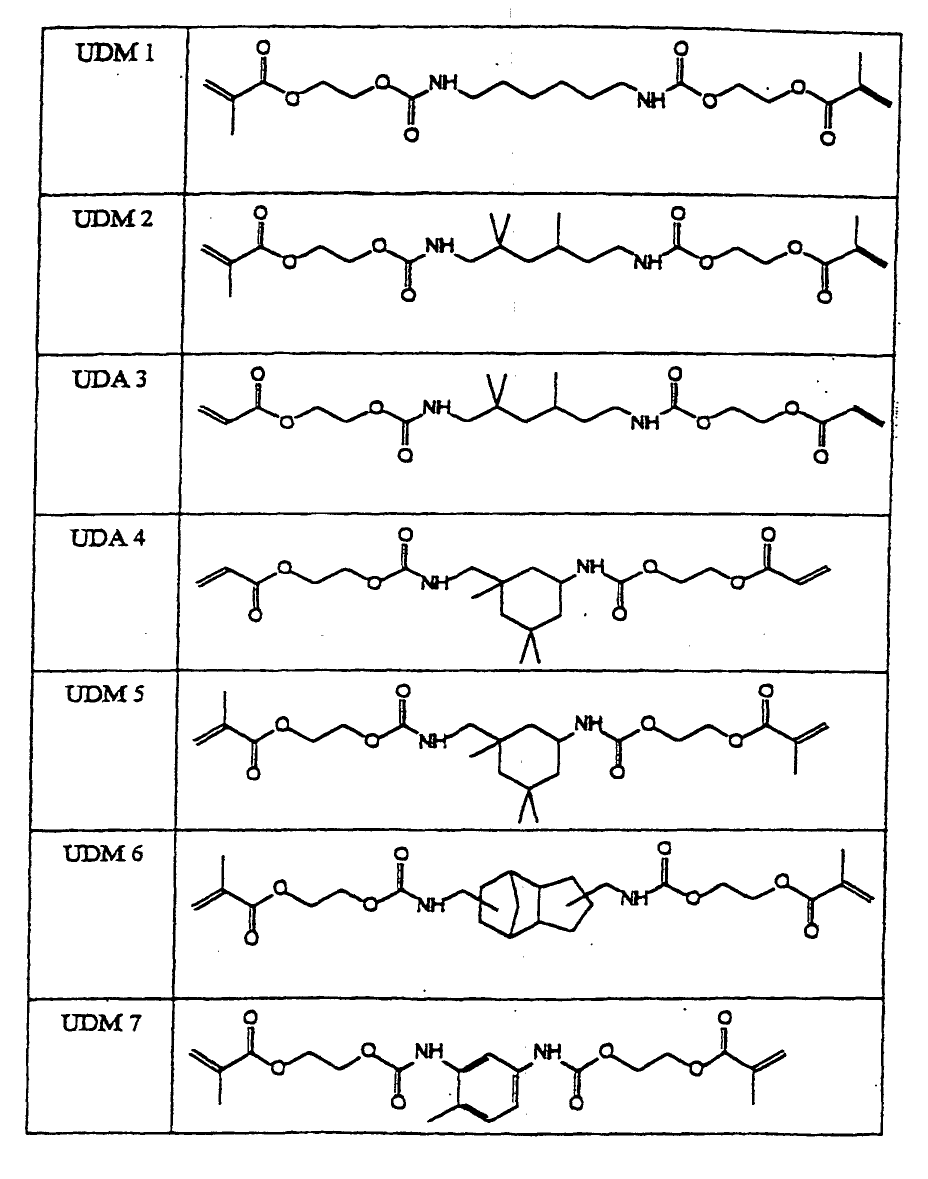

Die erfindungsgemäße Zubereitung enthält 2,5 bis 40 Gew.-%, vorzugsweise 5 - 25 Gew.-%, Methacryloyloxy-Gruppen aufweisende Ester aromatischer Tri- oder Tetracarbonsäuren. Anstelle der Tri- oder Tetracarbonsäuren können auch die entsprechenden Derivate eingesetzt werden, bei denen zwei benachbarte Carbonsäuregruppen eine Anhydridgruppe bilden. Als geeignete Methacryloyloxy-Gruppen aufweisende Ester aromatischer Tri- oder Tetracarbonsäuren seien die folgenden Verbindungen beispielhaft aufgeführt:

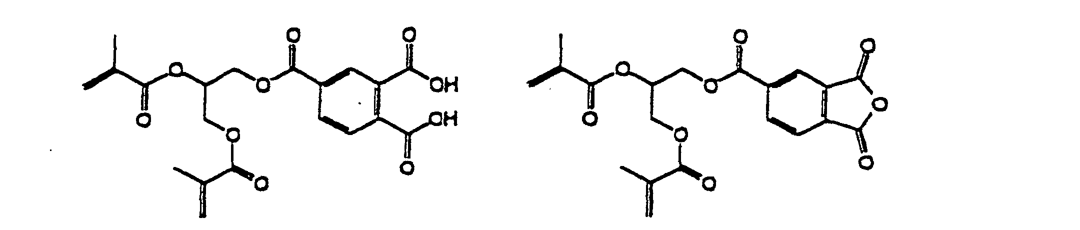

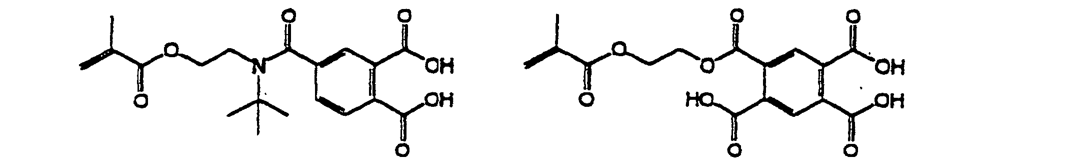

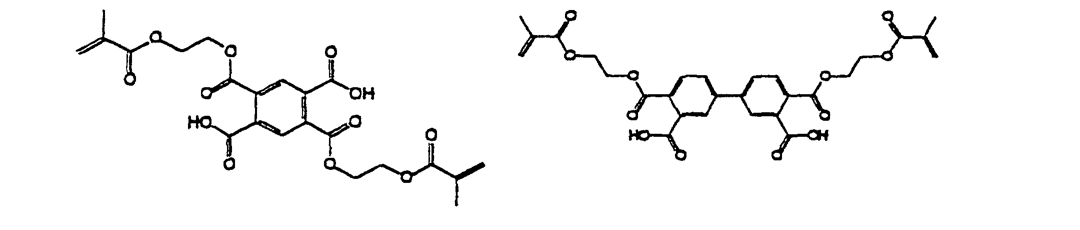

Besonders gut geeignet sind die in der Fachliteratur als "4-MET" und "4-META" bezeichneten Derivate der Trimellitsäure der Formeln:

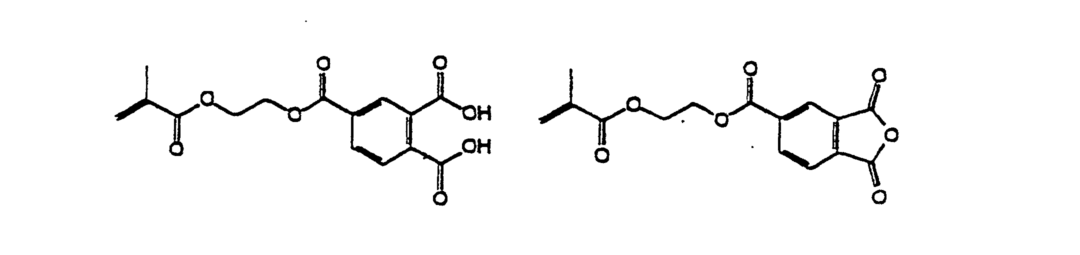

Flüchtige, mit Wasser mischbare Lösungsmittel sind vor allem solche mit einem Dampfdruck von mindestens 100 Torr bei Raumtemperatur. Bevorzugt sind aliphatische Alkohole mit ein bis vier C-Atomen, Aceton, 1,4-Dioxan und Tetrahydrofuran. Besonders bevorzugt sind Aceton und Ethylalkohol. Die Lösungsmittel sind in Mengen von 25 -75 Gew.-%, vorzugsweise 40- 60 Gew.-%, in der Zubereitung enthalten.

Fotoinitiatoren im Rahmen der vorliegenden Erfindung sind Radikalbildner, die unter der Einwirkung von Licht, beispielsweise UV-Licht, sichtbarem Licht oder Laserlicht, eine radikalische Polymerisation auslösen.

Diese Fotopolymerisationsinitiatoren sind an sich aus der Literatur bekannt. Vorzugsweise handelt es sich um Mono- oder Dicarbonylverbindungen, wie Benzophenon, Benzoin und dessen Derivate, insbesondere Benzoinmethylether, Benzil und Benzilderivate und andere Dicarbonylverbindungen, wie Diacetyl, 2,3-Pentandion und alpha-Diketoderivate des Norbomans und substituierter Norbomane, Metallcarbonyle, wie Pentacarbonylmangan, oder Chinone, wie 9,10-Phenanthrenchinon und Naphthochinon. Besonders bevorzugt ist Campherchinon.

Die erfindungsgemäße Zubereitung enthält im allgemeinen 0,01 bis 2,5 Gew.-%, bevorzugt 0,1 bis 0,5 Gew.-%, des Fotoinitiators, bezogen auf die erfindungsgemäße Zubereitung.

Es kann vorteilhaft sein, der erfindungsgemäßen Zubereitung Coaktivatoren zuzusetzen, die die Polymerisationsreaktion beschleunigen. Bekannte Beschleuniger sind beispielsweise Amine, wie p-Toluidin und Dimethyl-p-toluidin, Trialkylamine, wie Trihexylamin, Polyamine, wie N,N,N',N'-Tetraalkylalkylendiamine, Barbitursäure und Dialkylbarbitursäuren. Auch die Ester der 4-Dimethylaminobenzoesäure sind gut geeignete Coaktivatoren. Besonders gut geeignet sind Dimethylaminobenzolsulfonamide entsprechend DE-A-31 35 113.

Die Coaktivatoren werden im allgemeinen in einer Menge von 0,02 bis 4 Gew.-%, bevorzugt 0,2 bis 1 Gew.-%, bezogen auf die erfindungsgemäße Zubereitung, eingesetzt.

Die erfindungsgemäße Zubereitung kann neben Urethandimethacrylat, Methacryloyloxy-Gruppen aufweisendem Ester aromatischer Tri- oder Tetracarbonsäuren, Lösungsmittel, Fotoinitiator und Coaktivator gegebenenfalls weitere (Meth)acrylsäureester als Comonomere enthalten. Bevorzugt seien Ester der (Meth)acrylsäure mit 1- bis 5-wertigen Alkoholen mit 2 bis 30 Kohlenstoffatomen genannt. Außerdem seien Derivate des Tricyclodecans (EP-A-0 023 686) und Umsetzungsprodukte aus Polyolen, Diisocyanaten und Hydroxyalkylmethacrylaten (DE-A-37 03 120, DE-A-37 03 080 und DE-A-37 03 130) genannt.

Besonders bevorzugt als (Meth)acrylsäureester wird das sogenannte Bis-GMA der Formel

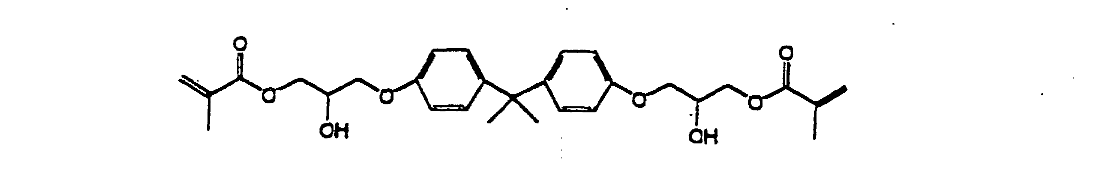

Eine besonders gute Elastizität der durch Polymerisation ausgehärteten Schicht der Zubereitung wird erzielt, wenn die Zubereitung zusätzlich Polyethylenglykoldi(meth)acrylate, insbesondere Polyethylenglykoldi(meth)acrylate mit einem Molekulargewicht von 200 - 2000, enthält. Geeignete Zusätze zur Verbesserung der elastischen Eigenschaften - sogenannte Elastifizierungsmittel - können auch aus Polyester(meth)acrylaten, Polyesterpolyurethan(meth)acrylaten und anderen Polyether(meth)acrylaten, wie Polybutylenglykoldimethacrylaten (EP 0 438 628) bestehen. Die Elastifizierungsmittel werden in Mengen von 0,5 bis 30 Gew.-%, vorzugsweise von 1 bis 15 Gew.-%, bezogen auf die Zubereitung, angewendet.

Es wurde gefunden, daß die an sich sehr hohen Bindungsfestigkeiten an Dentin durch einen Anteil an Füllstoff noch weiter gesteigert werden können. Geeignete Füllstoffe sind beispielsweise Bergkristall, Kristobalit, Quarz, Quarzglas, hochdisperse Kieselsäuren, Aluminiumoxid und Glaskeramiken. Die mittlere Teilchengröße der Füllstoffe liegt im allgemeinen im Bereich von 5 - 2000 nm, vorzugsweise im Bereich von 10-100 nm. Besonders gut geeignete Füllstoffe sind hochdisperse Kieselsäuren, die beispielsweise durch Flammhydrolyse gewonnen werden können. Besonders wirksam sind Zusätze in einer Menge von 5 bis 20 Gew.-%, bezogen auf die Zubereitung.

Die Füllstoffe werden vorzugsweise vorbehandelt, beispielsweise mit Silanisierungsmitteln aus Organosilicium-Verbindungen (Progress in Organic Coatings 11, 297-308 (1983). Ein bevorzugtes Silanisierungsmittel ist 3-Methacryloyloxy-propyl-trimethoxysilan.

Die Zubereitung gemäß der Erfindung kann weiterhin übliche Zusätze, wie Stabilisatoren, Inhibitoren und Lichtschutzmittel, enthalten.

Die erfindungsgemäße Zubereitung kann in einfacher Weise durch Mischen der einzelnen Komponenten hergestellt werden.

Die erfindungsgemäße Zubereitung eignet sich als Adhäsivkomponente zur Behandlung von Keramik, Metallen und Metall-Legierungen und wird vorzugsweise zur Behandlung der Zahnhartsubstanz und von Dentallegierungen verwendet.

In einer besonderen Ausführungsform konditioniert man die Zahnhartsubstanz vor der Behandlung mit der erfindungsgemäßen Zubereitung mit einer Konditionierungsflüssigkeit, die einen pH-Wert im Bereich von 0,1 bis 3,5 aufweist. Die Konditionierungsflüssigkeit enthält im allgemeinen Säuren mit einem pKs-Wert kleiner als 5. Folgende Säuren können z.B. in der Konditionierungsflüssigkeit enthalten sein: Phosphorsäure, Salpetersäure, Brenztraubensäure, Zitronensäure, Oxalsäure, Ethylendiamintetraessigsäure, Essigsäure, Weinsäure und Äpfelsäure. Weiterhin kann die Konditionierungsflüssigkeit Stoffe aus der Gruppe der Polyethylenglykole und Metallhydroxide enthalten. Insbesondere können die oben aufgeführten mehrbasigen Säuren auch als teilweise Metallsalze eingesetzt werden, solange freie Säurefunktionen verbleiben. Bevorzugt wird die Behandlung mit wäßriger Phosphorsäure. Geeignete Konzentrationen der Phosphorsäure sind 10 - 60 Gew.-%, vorzugsweise 20 - 40 Gew.-%. Die Konditionierungsflüssigkeit kann zur Einstellung einer geeigneten Konsistenz Verdickungsmittel, wie z.B. Kieselsäure, enthalten.

Die Anwendung der erfindungsgemäßen Zubereitung kann beispielsweise wie folgt durchgeführt werden:

Bei einer Zahnrestaurierung trägt man nach mechanischer Reinigung der Zahnoberfläche zuerst die Konditionierungsflüssigkeit auf, läßt eine kurze Zeit (beispielsweise 60 Sekunden) einwirken, spült die Zahnoberfläche mit Wasser und trocknet sie. Dann trägt man die erfindungsgemäße Zubereitung, beispielsweise mit einem kleinen Pinsel, in einer oder mehreren Schichten auf, trocknet im Luftstrom und bestrahlt mit einer handelsüblichen Polymerisationslampe. Danach trägt man das eigentliche Füllungsmaterial, beispielsweise ein im Dentalbereich übliches Kunststoff-Füllungsmaterial, auf.

Zur näheren Erläuterung werden im Folgenden Beispiele für die erfindungsgemäße Zubereitung (Beispiel 1) und die Prüfung ihrer Wirksamkeit a) durch Bestimmung der Scherbindungsfestigkeit von Kunststoff-Füllungen auf Dentin und Schmelz (Beispiel 2) und auf Dentallegierungen (Beispiel 4) und b) durch Bestimmung der Kavitätenadaptation (Beispiel 3) nach Vorbehandlung mit der Zubereitung beschrieben.

### Beispiel 1

Die folgenden Zubereitungen werden durch intensives Vermischen der Bestandteile erzeugt.

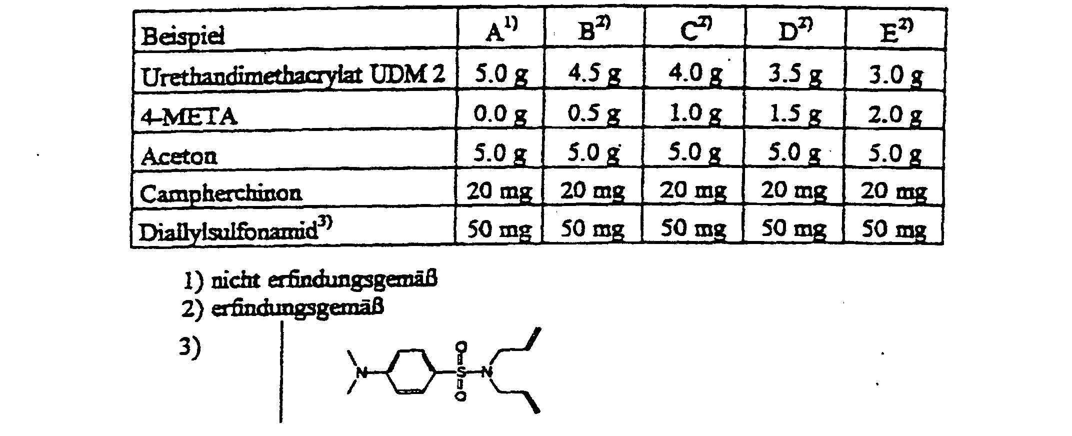

### Beispiel 2

### Bestimmung der Scherbindungsfestigkeit an Dentin

Die Wirksamkeit der in Beispiel 1 beschriebenen Zubereitungen wird geprüft durch Bestimmung der Scherbindungsfestigkeit auf Dentin. Es werden menschliche Zähne benutzt, die für max. drei Monate nach der Extraktion in 1 % Chloraminlösung aufbewahrt worden waren. Vor der Verwendung im Bindungstest werden die Zähne nach sorgfältiger Reinigung unter fließendem Wasser für mindestens drei und höchstens zehn Tage in physiologischer Kochsalzlösung gelagert. Am Tage vor der Verwendung im Bindungstest werden die Zähne einzeln auf einer Approximalseite liegend mit Epoxidharz (Lekutherm X20, Härter T3) in zylindrische Gummiformen von 25 mm Durchmesser und 12 mm Höhe eingebettet. Die Zähne werden durch Naßschleifen mit SiC-Papieren der Körnungen 240, 320, 400 und schließlich 600 soweit beschliffen, daß eine ausreichend große, schmelznahe Dentinfläche zur Anbindung eines Kunststoff-Zylinders mit 3,5 mm Durchmesser freiliegt. Nach Abspülen mit entionisiertem Wasser und Trocknung im Luftstrom wird 30 Sekunden lang das Konditionierungsmittel Gluma® CPS Gel (20 % H3PO4, Bayer AG) mit einem Wattepellet unter reibender Bewegung aufgetragen, mit Wasser sorgfältig abgespült und durch Abtupfen mit Zellstoff oberflächlich von Wasser befreit (feuchte Technik). Auf die konditionierte Dentinfläche werden die Zubereitungen aus Beispiel 1 mit einem Pinsel in drei Schichten aufgetragen, im Druckluftstrom getrocknet und mit dem Lichtgerät TRANSLUX® CL (Kulzer) 20 Sekunden lang bestrahlt. Die so vorbehandelten Proben werden dann mittels einer Einspannvorrichtung unter zweigeteilten zylindrischen Teflonformen (3,5 mm Durchmesser, 1 mm Höhe) festgeklemmt. Danach wird das Kunststoff-Füllungsmaterial PEKAFILL® U (Bayer AG) mit einer Spritze in die Teflonformen gefüllt, mit einem 02-undurchlässigen Strip abgedeckt und mit der Lichtgerät TRANSLUX® CL 60 Sekunden lang bestrahlt. Unmittelbar anschließend werden die Teflonformen abgenommen und die Proben für 24 Stunden in 37°C warmem Wasser gelagert bis zur Einleitung der Scherbelastung. Dazu werden die mit den Kunststoff-Zylindern versehenen Proben in einer Universalprüfmaschine mit Hilfe eines Druckstempels parallel zu und dicht an der Oberfläche des eingebetteten Zahnes unter einer Vorschubgeschwindigkeit von 1mm/Minute bis zur Trennung des Zylinders vom Zahn belastet. Die Scherbindungsfestigkeit ist der Quotient aus Bruchkraft und Bindungsareal und wird jeweils an 5 Proben bestimmt und als deren Mittelwert in der Tabelle I angegeben.

### Bestimmung der Scherbindungsfestigkeit an Schmelz

Für die Bestimmung der Scherbindungsfestigkeit an mit den im Beispiel 1 beschriebenen Zubereitungen behandeltem Schmelz werden extrahierte menschtiche Zähne mit intakter labialer Schmelzfläche in Epoxidharz eingebettet und mit nassem SiC-Papier der Körnung 240 bis 600 angeschliffen, um eine plane, periphere Schmelzoberfläche für den Bindungstest freizulegen. Auf die Schmelzoberfläche wird das Konditionierungsmittel Gluma® CPS Gel aufgetragen, das nach 30 Sekunden Einwirkzeit sorgfältig mit entionisiertem Wasser abgespült wird. Die Trocknung erfolgt nur oberflächlich mit einem schwachen Druckluftstrahl, bis die behandelte Oberfläche kreidig weiß erscheint. Alle weiteren Arbeitsschritte sind identisch mit den vorstehend beschriebenen zur Bestimmung der Scherbindungsfestigkeit an Dentin. Die Werte für die Scherbindungsfestigkeit an Schmelz werden in der Tabelle I angegeben.

**Tabelle I**

| Zubereitung | Scherbindungsfestigkeit an Dentin (MPa) | Scherbindungsfestigkeit an Schmelz (MPa) |
|---|---|---|
| Beispiel 1A¹⁾ | 15.1 ± 4.3 | 30.3 ± 3.3 |
| Beispiel 1B | 20.4 ± 2.6 | 33.0 ± 3.9 |
| Beispiel 1C | 22.0 ± 4.6 | 34.5 ± 5.2 |
| Beispiel 1D | 21.9 ± 4.5 | 33.0 ± 2.0 |
| Beispiel 1E | 20.4 ± 4.2 | 28.0 ± 5.8 |

| | | |
|---|---|---|
| 1) nicht erfindungsgemäß | | |

### Beispiel 3

### Kavitätenadaptation

Eine weitere Aussage über die Wirksamkeit eines Dentaladhäsivs läßt sich aufgrund von Untersuchungen des Randes von Zahnfüllungen machen, die in mit dem Dentaladhäsiv vorbehandelte Dentinkavitäten gelegt werden. In der Zahnheilkunde werden in der Regel intradentale Defekte (von Zahnhartsubstanz umgebene Kavitäten) mit Füllungsmaterialien gefüllt. Dabei entstehen besonders bei Kunststoff-Füllungsmaterialien aufgrund der bei der Härtung auftretenden Schrumpfung Wand-zu-Wand Kontraktionsspannungen, die zur Ablösung der Kunststoff-Füllung von der Kavitätenwandung und damit zu Bildung von Randspalten im Bereich des Füllungsrandes führen können. Das Verhalten der Zahnfüllungen an der Kavitätenwandung wird als Kavitätenadaptation bezeichnet. Dabei bedeuten nicht grundsätzlich Materialien mit hoher Bindungsfestigkeit gute und Materialien mit geringer Bindungsfestigkeit schlechte Kavitätenadaptation.

Zur Prüfung der Kavitätenadaptation werden extrahierte, menschliche Molaren (Mahlzähne) verwendet, die für maximal drei Monate nach Extraktion in 1 % Chloraminlösung gelagert worden sind. Die Zähne werden auf einer intakten Approximalseite mit nassen SiC-Papieren der Körnungen 240, 320, 400 und 600 bis zur Freilegung ausreichend großer, schmelznaher Dentinflächen plangeschliffen. Ausgehend von den plangeschliffenen Dentinflächen werden in das Dentin der Zähne unter Wasserkühlung mit feinkörnigen, diamantierten Präparationsinstrumenten auf einem mit einem Mikromotor betriebenen zahnärztlichen Handstück zylinderförmige Kavitäten mit einem Durchmesser von ca. 3,5 mm und einer Tiefe von 1,5 mm präpariert. Der Kavitätenkantenwinkel beträgt 90°. Die Vorbehandlung der Kavitäten erfolgt mit dem Konditionierungsmittel Gluma® CPS Gel für 30 Sekunden. Anschließend wird das Gel mit Wasser sorgfältig abgespült und jede Kavität mit einem Wattepellet oberflächlich getrocknet. Wie bei den Bindungstests werden dann die im Beispiel 1 beschriebenen Zubereitungen mit einem Pinsel in zwei bis drei Schichten auf die Kavitätenwandungen aufgetragen. Die nach dem Entfernen des Lösungsmittels mit Druckluft verbleibenden dünnen Schichten werden 20 Sekunden lang mit Licht bestrahlt (Translux® CL), bevor die Kavität mit dem Kunststoff-Füllungsmaterial Pekafill® U gefüllt, mit einem lichtdurchlässigen Strip abgedeckt und 60 Sekunden lang mit Licht (Translux® CL) bestrahlt werden. Die mit den Füllungen versehenen Zähne werden sofort für 15 Minuten in entionisiertes Wasser (23° C) gelegt. Anschließend wird mit nassem SiC-Papier (Körnungen 600 und 4000) der Füllungsüberschuß entfernt, bis der Rand der Kavitäten freiliegt. Unmittelbar danach erfolgt im Auflichtmikroskop bei 500facher Vergrößerung eine Inspektion des Randes. Sofern sich zwischen den Füllungen und den Kavitätenwandungen Randspalten gebildet haben, wird die maximale Breite der Randspalten mit Hilfe eines Okular-Schraubenmikrometers bestimmt. In der Tabelle II werden die Breite der Randspalten (Mittelwerte von je 6 Proben) und die Anzahl der Zahnfüllungen ohne Randspalt zwischen Kavitätenwandung und Füllung von je 6 Proben angegeben.

**Tabelle II**

| Zubereitung | Randspalt [µm] | Anzahl spaltfreier Füllungen |
|---|---|---|
| Beispiel 1A¹⁾ | 3.03 ± 1.78 | 1 |
| Beispiel 1B | 1.68 ± 2.02 | 3 |
| Beispiel 1C | 2.82 ± 2.33 | 2 |
| Beispiel 1D | 0.78 ± 1.45 | 4 |
| Beispiel 1E | 0.0 | 6 |

| | | |
|---|---|---|
| 1) nicht erfindungsgemäß | | |

### Beispiel 4

Bestimmung der Scherbindungsfestigkeit an der Dentallegierung Levochrom Zur Prüfung der Bindungsfestigkeit von Kunststoff-Füllungsmaterialien an mit den erfindungsgemäßen Zubereitungen vorbehandelten Dentallegierungen wird die CrCo-Gußlegierung Levochrom (Bayer AG) eingesetzt. Aus der Legierung werden würfelförmige Proben gegossen und wie die extrahierten Zähne in Epoxidharz eingebettet. Die Proben werden mit SiC-Papier (240 - 600) angeschliffen, mit 50 um Edelkorund abgestrahlt und 5 Minuten in entionisiertem Wasser im Ultraschallbad gereinigt. Nach dem Trocknen der Proben mit Druckluft werden die im Beispiel 1 beschriebenen Zubereitungen in zwei Schichten aufgetragen. Anschließend - nach Abdampfen des Lösungsmittel - werden die so vorbehandelten Proben 20 Sekunden lang mit dem Lichtgerät (Translux® CL) bestrahlt und entsprechend den Bindungstests an Dentin und Schmelz durch Fotopolymerisation mit einem Zylinder (3,5 mm Ø, 1,5 mm hoch) aus dem Kunststoff-Füllungsmaterial verbunden. Die Bestimmung der in der Tabelle III angegebenen Scherbindungsfestigkeit erfolgt nach 24stündiger Lagerung der Proben in 37° C Wasser, wie in Beispiel 2 beschrieben.

**Tabelle III**

| Zubereitung | Scherbindungsfestigkeit an Levochrom (MPa) |
|---|---|
| Beispiel 1A¹⁾ | 10.8 ± 1.9 |
| Beispiel 1B | 17.3 ± 1.6 |
| Beispiel 1C | 16.2 ± 2.2 |
| Beispiel 1D | 14.8 ± 4.8 |
| Beispiel 1E | 11.1 ± 1.1 |

| | |
|---|---|
| 1) nicht erfindungsgemäß | |

## Patentansprüche

1. Hydroxyethylmethacrylat-freie Zubereitung zur Verwendung als Adhäsivkomponente, **dadurch gekennzeichnet, dass** sie aus
a) 10 - 47,5 Gew.-% Urethandi(meth)acrylat,
b) 2,5 - 40 Gew.-% Methacryloyloxy-Gruppen aufweisendem Ester aromatischer Tri- oder Tetracarbonsäuren oder der entsprechenden Anhydride,
c) 25 - 75 Gew.-% eines flüchtigen, mit Wasser mischbaren Lösungsmittels
d) 0,01 -2,5 Gew.-% Fotoinitiator und
e) 0 - 40 Gew.% an sich bekannter Zusätze besteht.

2. Zubereitung nach Anspruch 1, **dadurch gekennzeichnet, dass** sie
e) 5 - 20 Gew.-% Füllstoff enthält.

3. Zubereitung nach Anspruch 2, **dadurch gekennzeichnet, dass** sie als Füllstoff hochdisperse Kieselsäure mit einer mittleren Teilchengröße von 5 - 2000 nm enthält.

4. Zubereitung nach einem der Ansprüche 1 bis 3, **dadurch gekennzeichnet, dass** sie
b) 4-MET oder 4-META enthält.

5. Zubereitung nach einem der Ansprüche 1 bis 4 zur Verwendung bei der Behandlung von Zahnhartsubstanz.

6. Verwendung der Zubereitung nach einem der Ansprüche 1 bis 4 zur Behandlung von Dentallegierungen.

## Claims

1. Hydroxyethyl methacrylate-free formulation for use as an adhesive component, **characterized in that** it consists of
a) 10 - 47.5% by weight of urethane di(meth)acrylate,
b) 2.5 - 40% by weight of an ester of aromatic tri-or tetracarboxylic acids or of the corresponding anhydrides, which ester has methacryloyloxy groups,
c) 25 - 75% by weight of a volatile, water-miscible solvent,
d) 0.01 - 2.5% by weight of photoinitiator and
e) 0 - 40% by weight of additives known per se.

2. Formulation according to Claim 1, **characterized in that** it contains
e) 5 - 20% by weight of filler.

3. Formulation according to Claim 2, **characterized in that** it contains, as a filler, colloidal silica having a mean particle size of 5 - 2000 nm.

4. Formulation according to any of Claims 1 to 3, **characterized in that** it contains
b) 4-MET or 4-META.

5. Formulation according to any of Claims 1 to 4 for use in the treatment of hard tooth substance.

6. Use of the formulation according to any of Claims 1 to 4 for the treatment of dental alloys.

## Revendications

1. Préparation sans méthacrylate d'hydroxyéthyle destinée à être utilisée comme composant adhésif, **caractérisée en ce qu'**elle consiste en :
a) 10 - 47,5 % en masse de di(méth)acrylate d'uréthane,
b) 2,5 - 40 % en masse d'ester comportant des groupes méthacryloyloxy d'acides tri- ou tétracarboxyliques aromatiques ou des anhydrides correspondants,
c) 25 - 75 % en masse d'un solvant volatil miscible à l'eau,
d) 0,01 - 2,5 % en masse de photoinitiateur et
e) 0 - 40 % en masse d'additifs connus en soi.

2. Préparation selon la revendication 1, **caractérisée en ce qu'**elle contient
e) 5 - 20 % en masse de charge.

3. Préparation selon la revendication 2, **caractérisée en ce qu'**elle contient comme charge de l'acide silicique hautement dispersé ayant une taille de particule moyenne de 5 - 2 000 nm.

4. Préparation selon l'une des revendications 1 à 3, **caractérisée en ce qu'**elle contient
b) du 4-MET ou du 4-META.

5. Préparation selon l'une des revendications 1 à 4 destinée à être utilisée dans le traitement de la substance dure des dents.

6. Utilisation de la préparation selon l'une des revendications 1 à 4 pour le traitement d'alliages dentaires.
